# EUROPEAN PATENT SPECIFICATION

(11) **EP 2 004 593 B1**
(45) Date of publication and mention of the grant of the patent: **21.03.2012**
(21) Application number: 07723633.9
(22) Date of filing: 27.03.2007
(51) Int. Cl.: C07C 233/25, A61K 31/167, A61P 3/04

(54) **IMPROVED CRYSTALLINE MATERIAL**
VERBESSERTES KRISTALLINES MATERIAL
MATÉRIAU CRISTALLIN AMÉLIORÉ

(30) Priority: 28.03.2006 GB 0606201
(43) Date of publication of application: 24.12.2008
(60) Divisional application: 11171176.8
(73) Proprietor: KARO BIO AB, 141 57 Huddinge (SE); Bristol-Myers Squibb Company, Princeton NJ 08543-4000 (US)
(72) Inventor: CHIDAMBARAM, Ramakrishnan, Santa Clara, CA 95054 (US); GARG, Neeraj, S-141 57 Huddinge (SE); RASMUSON, Ake, S-100 44 Stockholm (SE); GRACIN, Sandra, Stevenage SG1 3LU (GB)
(74) Representative: Brady, Paul Andrew
(86) International application number: PCT/EP2007/002687
(87) International publication number: WO 2007/110225

(56) References cited:
- WO-A-01/60784
- WO-A-01/72692
- WO-A-01/85670
- WO-A-02/094319
- WO-A-2004/067482
- US-A1- 2003 157 671

## Description

This invention relates to an improved crystalline material and its use in the treatment of conditions associated with thyroid disfunction.

WO 01/60784 relates to novel compounds which are agonists of the thyroid beta receptor. Example 1 describes the synthesis of 3-[[3,5-dibromo-4-[4-hydroxy-3-(1-methylethyl)-phenoxy]-phenyl]-amino]-3-oxopropanoic acid. This compound is hereinafter referred to as Compound I, and has the formula: 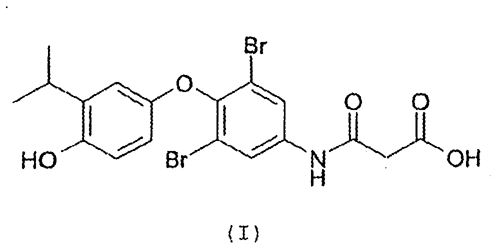 Synthesis of Compound I is rather difficult, and US 2003/0157671 relates to an improved method of synthesis.

This method, and also the method of Example 1 of WO 01/60784, leads to a specific crystalline form, referred to herein as the N-1 crystal form. Unfortunately, the N-1 crystal form has proved to be unstable.

It has now been found that Compound I can be synthesised in two novel crystalline forms which have improved properties compared with the same compound in other forms.

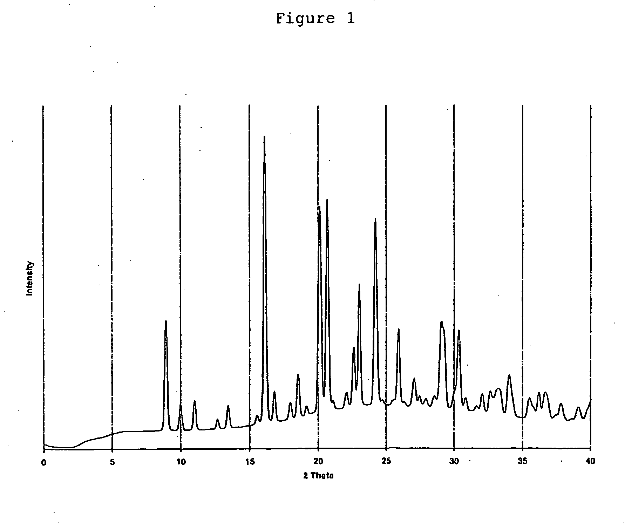

The present invention provides 3-[[3,5-dibromo-4-[4-hydroxy-3-(1-methylethyl)-phenoxy]-phenyl]-amino]-3-oxopropanoic acid (Compound I) in a crystalline form which is characterised by a powder X-ray diffraction (XRD) pattern having major peaks at 2θ = 16.1±0.2, 20.1±0.2, 20.7±0.2, and 24.2±0.2. This novel crystalline material of the invention will be referred to as Form N-6 throughout this Specification. Other significant but less prominent peaks in the XRD may be found at 2θ = 8.9±0.2, 23.0±0.2, 25.9±0.2, 29.1±0.2, 29.4±0.2, and 30.3±0.2. A typical complete X-ray diffraction pattern is shown in Figure 1. Table 1. gives those peaks having an intensity of greater than 10% (normalised to the most intense peak).

| Table 1 XRD of Form N-6 | |
|---|---|
| 2-Theta | I% |
| 8.94 | 41.7 |
| 10.01 | 10.1 |
| 11.00 | 11.8 |
| 16.12 | 100.0 |
| 16.82 | 11.7 |
| 18.54 | 16.2 |
| 20.12 | 75.4 |
| 20.66 | 73.9 |
| 22.59 | 21.2 |
| 23.00 | 44.6 |
| 24.21 | 69.1 |
| 25.93 | 23.2 |
| 29.14 | 44.5 |
| 29.37 | 23.0 |
| 30.33 | 25.0 |

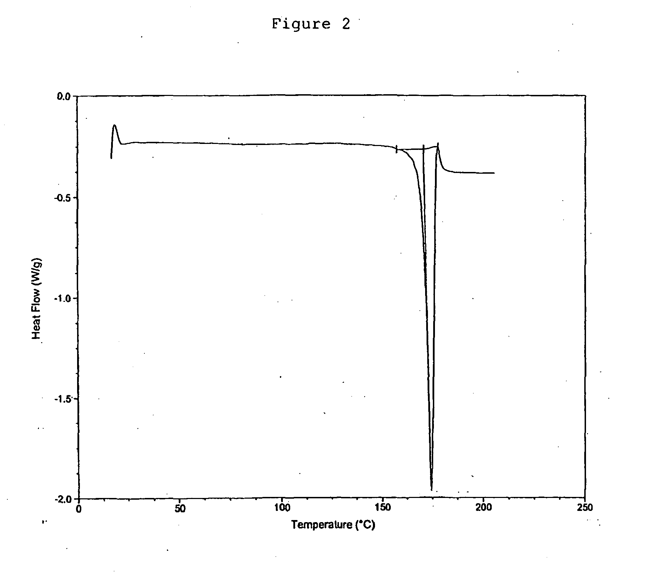

An additional way of characterising the novel crystalline material Form N-6 of the invention is by its differential scanning calorimetry (DSC) thermogram. Typically the material has a DSC thermogram which exhibits a single endotherm with a maximum at 174±6°C (and an extrapolated onset of 170±6°C). Complete melting occurs by 182±6°C, and heat of fusion is about 88J/g. A typical DSC trace is shown in Figure 2.

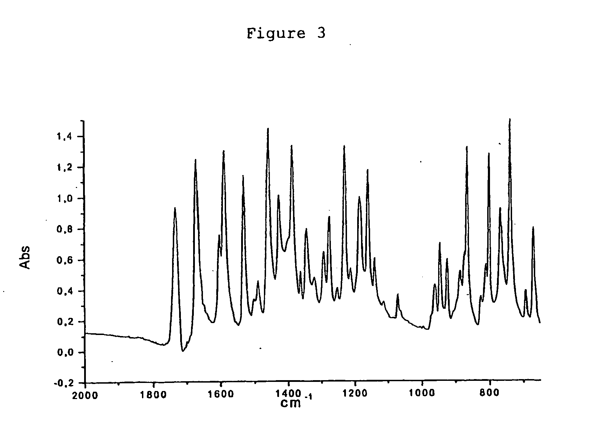

Further, the novel crystalline material Form N-6 of the invention may be characterised by its Fourier Transform infra-red (FTIR) spectrum. A typical FTIR spectrum is shown in Figure 3.

Naturally, the exact details of any XRD, DSC or FTIR pattern will depend upon a number of factors, for example the instrument used and the degree of purity of the material. DSC's in particular, may be instrument dependent. The numbers quoted throughout this Specification refer to the use of a TA instruments MDSC-2920 machine.

Preferably Form N-6 is provided according to the invention at a level of purity in which at least 90%, especially at least 95%, most preferably all, of the Compound I present, is Form N-6.

Compound I can itself be synthesised as described in either WO 01/60784 or US 2003/0157671. Resulting Compound I having a different physical structure from Form N-6 may then be converted into Form N-6 by a method which comprises preparing a solution of Compound I in a suitable solvent, and seeding with crystals of either Form N-5 (see below) or Form N-6, under conditions such that Form N-6 is obtained. Alternatively, a solution of Compound I may be prepared *in situ* and this solution used directly to obtain crystals of From N-6. It is a characteristic of Compound I that the thermodynamic equilibrium between Forms N-5 and N-6 is finely balanced. Thus, at a temperature of less than about 50°C, crystals of either Form N-5 or Form N-6 may be used to seed a solution of Compound I to prepare crystals of N-6. Suitable solvents in this context include, for example, water, alcohols, for example methanol, ethanol, isopropanol or hexanol, ketones, for example acetone, DMSO, esters, for example ethyl acetate, acids, for example acetic acids, nitriles, for example acetonitrile, amides, for example DMF, nitromethane or nitroethane, or hydrocarbons, for example toluene or hexane; or mixtures thereof. Form N-5 may be prepared as described below.

When crystals of Form N-6 are already available, one preferred method of preparing further Form N-6 comprises preparing a solution of Compound I in an alcohol or alcohol mixture, for example an ethanol/isopropanol mixture; adding water; optionally filtering the resulting solution; and seeding the resulting solution with crystals of Form N-6 to produce further crystals of Form N-6.

Although Form N-5 is generally very stable, under certain conditions it may convert to Form N-6. Accordingly, a further method of preparing Form N-6 comprises slurrying Form N-5 in an alcohol/water mixture for a sufficient period of time to allow conversion to Form N-6. An extended period of time, typically from 6 to 10 days, is generally required.

Form N-6 has been found to have a number of advantageous properties. In particular, it is highly stable, being heat stable up to at least 170°C, and is non-hygroscopic up to a relative humidity of 95%.

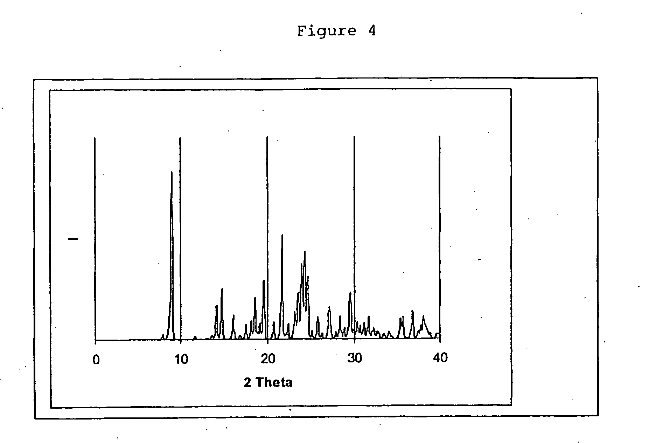

As mentioned above, 3-[[3,5-dibromo-4-[4-hydroxy-3-(1-methylethy)-phenoxy]-phenyl]-amino]-3-oxopropanoic acid (Compound I) may also exist in a crystalline form which is characterised by an X-ray diffraction (XRD) pattern having major peaks at 2θ = 9.0±0.2, 14.7±0.2, 19.6±0.2, 21.6±0.2, and 24.3±0.2. This second novel crystalline material will be referred to as Form N-5 throughout this Specification. Other significant but less prominent peaks may be found at 2θ = 14.1±0.2, 16.1±0.2, 18.6±0.2, 23.1±0.2, 23.5±0.2, 24.7±0.2, and 29.4±0.2. A typical complete X-ray diffraction pattern is shown in Figure 4. Table 2 gives those peaks having an intensity of greater than 8% (normalised to the most intense peak).

| Table 2 XRD of Form N-5 | |
|---|---|
| 2-Theta | I% |
| 8.65 | 8.0 |
| 9.00 | 100.0 |
| 14.11 | 13.6 |
| 14.72 | 31.3 |
| 16.12 | 14.9 |
| 17.56 | 8.0 |
| 18.59 | 15.0 |
| 19.09 | 10.9 |
| 19.56 | 23.1 |
| 21.61 | 54.1 |
| 23.14 | 14.5 |
| 23.51 | 18.6 |
| 23.86 | 25.1 |
| 24.29 | 31.7 |
| 24.66 | 17.9 |
| 27.11 | 8.9 |
| 29.40 | 18.5 |
| 30.29 | 12.3 |

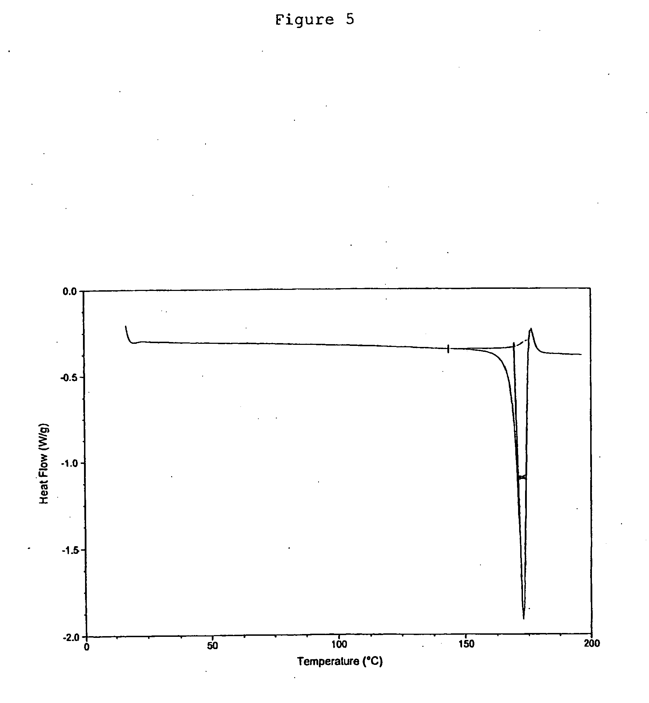

An additional way of characterising the novel crystalline material Form N-5 of the invention is by its differential scanning calorimetry (DSC) thermogram. Typically the material has a DSC thermogram which exhibits an endotherm with a maximum at 173±6°C (and an extrapolated onset of 176°C). The heat of fusion is about 81J/g. A typical DSC trace is shown in Figure 5.

Naturally, the exact details of any XRD or DSC pattern will depend upon a number of factors, for example the instrument used and the degree of purity of the material.

Compound I can itself be synthesised as described in either WO 01/60784 or US 2003/0157671. Resulting Compound I having a different physical structure from Form N-5 may then be converted into Form N-5 by a method which comprises preparing a solution of the Compound I in a suitable solvent, and recrystallising under conditions such that crystals of Form N-5 are produced. Alternatively, a solution of Compound I may be prepared in situ and this solution used directly to obtain crystals of Form N-5. Suitable solvents in this context include, for example, water, alcohols, for example methanol, ethanol, isopropanol or heanol, ketones, for example acetone, DMSO, esters, for example ethyl acetate, acids, for example acetic acids, nitriles, for example acetonitrile, amides, for example DMF, nitromethane or nitroethane, or hydrocarbons, for example toluene or hexane; or mixtures thereof. In one method of preparing form N-5, a solution of the Compound I in an alcohol is prepared, preferably at room temperature, and recrystallisation is achieved by the addition of water. Alternatively, a solution of Compound I in nitroethane may be prepared at elevated temperature, and recrystallisation achieved by cooling.

In a variant of the above process, a slurry process may be used to prepare Form N-5. For example, a solution of Compound I in a suitable solvent, for example an alcohol or an alcohol mixture, may be prepared, and water may be added to produce a suspension. The resulting slurry may then be stirred for a period of time sufficient for Form N-5 to be produced.

Under certain conditions, Form N-5 can convert into Form N-6, and therefore care needs to be taken to minimise this conversion when preparing Form N-5. For example, extended contact (i.e. contact over several days) with certain solvents, especially alcohol solvents, should be avoided.

Form N-5 has been found to have a number of advantageous properties. In particular, it is highly stable, being heat stable up to at least 170°C, and is non-hygroscopic up to a relative humidity of 95%. In general, it either does not convert to other forms, or only converts very slowly; for example, if slurried in the presence of an alcohol, it is stable for a number of days, but after very long periods of time, conversion into Form N-6 may occur.

It will be apparent that similar processes may be used to make Form N-6 and Form N-5. The nature of the product is determined by the precise reaction conditions, which may be readily determined by the skilled man with the aid of the Examples herein.

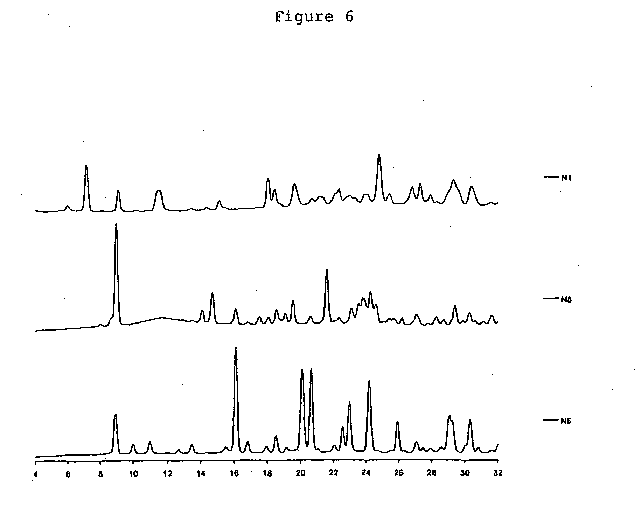

In contrast to Forms N-6 and N-5, the crystalline form produced by the process of US 2003/1057671, referred to herein as Form N-1, has major X-ray diffraction peaks at 2θ = 7.2±0.2, 9.1±0.2, 11.5±0.2, 18.1±0.2, 19.7±0.2, 24.8±0.2, 27.3±0.2, and 29.3±0.2, with additional significant but lower intensity peaks at 2θ = 18.5±0.2, 22.3±0.2, 26.8±0.2, and 30.4±0.2. A typical complete X-ray diffraction pattern of Form N-1 is shown in Figure 6, together with, for comparison, the XRDs of Form N-5 and Form N-6. The first peak for Form N-1 in Figure 6 is due to a contaminant, and should be discounted. Table 3 tabulates those peaks having an intensity of greater than 10%.

| Table 3 XRD of Form N-1 | |
|---|---|
| 7.17 | 91.9 |
| 9.13 | 43.5 |
| 11.51 | 40.8 |
| 15.15 | 17.8 |
| 18.07 | 60.8 |
| 18.46 | 36.4 |
| 19.67 | 46.0 |
| 20.73 | 13.7 |
| 21.24 | 16.4 |
| 22.32 | 31.6 |
| 23.02 | 20.0 |
| 24.03 | 20.3 |
| 24.82 | 100.0 |
| 25.43 | 20.7 |
| 26.82 | 36.3 |
| 27.32 | 43.1 |
| 27.92 | 18.7 |
| 29.30 | 49.4 |
| 30.41 | 35.7 |

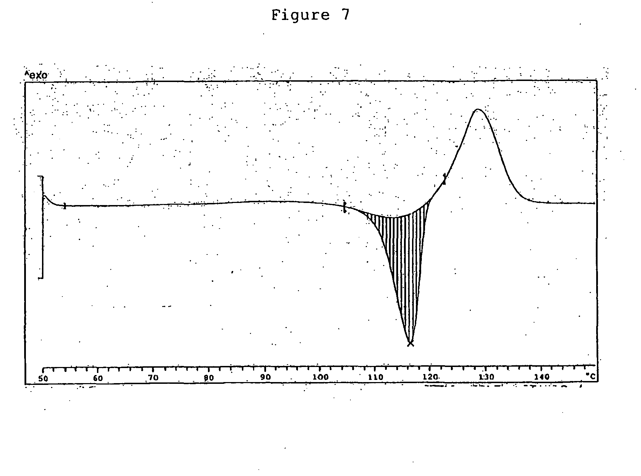

Typically Form N-1 has a DSC thermogram which exhibits a single endotherm with a maximum at 116±6°C (and an extrapolated onset of 111±6°C). The heat of fusion is about 58J/g. A typical DSC trace of Form N-1 is shown in Figure 7.

Form N-1 is less stable than either Form N-6 or Form N-5, being heat stable only up to around 125°C, and becoming hygroscopic at a relative humidity of 50%. Form N-1 converts into Form N-5 and/or Form N-6 (depending upon the exact conditions) on being dissolved or slurried in a number of common solvents, for example alcohols such as methanol, ethanol or isopropanol, nitromethane or nitroethane, and mixtures of alcohols, for example methanol, ethanol or isopropanol, with water.

The invention also provides a pharmaceutical composition comprising Form N-6 together with a pharmaceutically acceptable carrier. The pharmaceutical composition may be formulated for and administered to humans or other mammals by any suitable method. It is preferably formulated for oral administration in solid or liquid form, or for topical administration, for parenteral injection, or for rectal administration. Suitable forms of pharmaceutical composition, and methods of administration, include those described in WO 01/60784.

Pharmaceutical compositions according to the invention may also contain one or more other therapeutic agents. Suitable compounds for use in combination therapy are listed in WO 01/60784.

The crystalline material of the invention may be used for treating or preventing a condition associated with the thyroid receptor, by agonizing the effects, in particular the thyroid beta preceptor. Such conditions include obesity, hypercholesterolemia, atherosclerosis, depression, osteoporosis, hypothyroidism or subclinical hypothyroidism, non-toxic goiter, papillary or follicular thyroid cancer, glaucoma, cardiovascular disease, congestive heart failure, and skin disorders such as psoriasis, dermal atrophy, wound healing, keloids, stria, cellulite, roughened skin, actinic skin damage, lichen planus, ichtyosis, acne, psoriasis, Dernier's disease, eczema, atopic dermatitis, chloracne, pityriasis and skin acarring. Further details may be found in WO 01/60784. accordingly, the present invention also provides the crystalline material of the invention or a pharmaceutical composition according to the invention, for use in therapy.

The dosage level required in any individual case will depend upon various factors including the route of administration and the severity of the condition being treated. Very generally, dosage levels of about 0.001 to 500, more preferably of about 0.01 to about 100, most preferably of about 0.01 to 25mg, such as 0.01 to 1 mg, of Compound I per person per day, are administered. If desired, the effective daily dose may be divided into multiple doses for purposes of administration, e.g. two to four separate doses per day.

The invention is illustrated by reference to the accompanying drawings, in which:
Figure 1 is an XRD of Form N-6.
Figure 2 is a differential scanning calorimetry trace of Form N-6.
Figure 3 is an FTIR of Form N-6.
Figure 4 is an XRD of Form N-5
Figure 5 is a differential scanning calorimetry trace of Form N-5.
Figure 6 is an XRD of Form N-1, together with XRDs of Form N-5 and Form N-6.
Figure 7 is a differential scanning calorimetry trace of Form N-1.

The following Examples illustrate the invention. X-ray powder diffraction was performed using an Rigaku Ru300 (Rigaku Corporation 4-14-4, Sendagaya, Shibuya-Ku, Tokyo 151-0051, JAPAN) The source of radiation was a rotating anode operated at 50 kV and 100 mA emitting CuKa radiation (1=1.54179 Å). Data was collected on an MAR345 image plate (Marresearch GmbH, Norderstedt (Hamburg), Germany). Analysis of data used Fit2D (v. 12.081 A P Hammersley, ESRF, BP 220, 38043 Grenoble, France) to reduce the diffraction rings and optimize the beam centre positions.

DSC traces were obtained using a 2920 MDSC V2.5F machine, by testing about 5-10mg of sample in an aluminium DSC crucible, with a temperature gradient of 10°C/min.

The following Examples illustrate the invention.

### Example 1: Preparation of Form N-6

### Example 1A: Method A

3-[[3,5-Dibromo-4-[4-hydroxy-3-(1-methylethyl)-phenoxy]-phenyl]-amino]-3-oxopropanoic acid (8.15 g, Form N-1, prepared as in US 2003/0157671), was dissolved in 60 ml of solvent (40ml ethanol+20ml water) by heating the solution to 30°C and keeping it at that temperature for 10 min. The solution was then cooled down to 25°C by 1°C/min, without nucleation occurring. Seeds (confirmed to be pure N-5 by DSC, IR and XRD) were added in this experiment as a slurry (0.1g slurried in 20ml water), very slowly at 200µl/min, during which the solution temperature was kept constant. After 1 hour the solution became cloudy. Additional water (about 30ml) was added at the same rate. Addition of water was then stopped since the solution became opaque and viscous. After 17 hours at 25°C, the solution was cooled down to 8°C by 0.33°C/min and filtered. Form N-6, whose identity was confirmed by XRD, was obtained.

### Example 1B: Method B

3-[[3,5-Dibromo-4-[4-hydroxy-3-(1-methylethyl)-phenoxy]-phenyl]-amino]-3-oxopropanoic acid (0.63 kg, Form N-1, prepared as in US 2003/0157671) was dissolved in ethanol denatured with isopropanol (2.77L) at 30°C. Demineralised water (1.39L) was added, maintaining the temperature at 30°C. The solution was filtered on a cartridge filter (0.3 mm) and the filter was rinsed with a mixture of ethanol denatured with isopropanol (0.38L) and demineralised water (0.19L). The resulting solution was cooled to 25°C. A suspension of Form N-6 (13g) in demineralised water (1.26L) was added in about 1.5h. The dropping funnel was rinsed with demineralised water (0.32L) which was added in about 45 min. Demineralised water (1.58L) was added in about 4h and the resulting suspension was stirred for about 15h. Demineralised water (0.79L) was added in about 4h and the suspension was cooled to 8°C in 3h and stirred for about 15h. The product was filtered and the cake was washed with demineralised water (0.95L). The wet cake was dried at a jacket temperature of 25°C for about 24h. 0.61 kg (yield 94%) of Form N-6 was obtained.

### Example 1C: Method C

Solid Compound I consisting mostly of Form N-5 prepared as in Example 2 below, was slurried in an ethanol:water mixture (1:5 vol) at 20°C for 8 days. Form N-6 was produced.

### Example 2: Preparation of Form N-5 (not according to the invention)

### Example 2A: Method A

3-[[3,5-Dibromo-4-[4-hydroxy-3-(1-methylethyl)-phenoxy]-phenyl]-amino]-3-oxopropanoic acid (100 mg, Form N-1, prepared as in US 2003/0157671) was dissolved in ethanol (300µl) at room temperature. Deionised water (400µL) was added under stirring, the solution was clear after addition of water. More water (100µL) was added and the solution became cloudy. Additional deionised water (100µl) was added and the mixture was stirred further for 2hrs at room temperature on a magnetic stirrer. The solution was filtered and the solid was dried with air flow to give 66 mg of Form N-5, identified by XRD.

### Example 2B: Method B

3-[[3,5-Dibromo-4-[4-hydroxy-3-(1-methylethyl)-phenoxy]-phenyl]-amino]-3-oxopropanoic acid (100mg, Form N-1, prepared as in US 2003/0157671) was dissolved in 2-propanol (500µl) at room temperature. Deionised water (1500µL) was added under stirring, and the mixture was stirred further for 2 hrs at room temperature on a magnetic stirrer. The solution was filtered and the resulting solid was dried with air flow to give 58mg of Form N-5, identified by XRD.

### Example 2C: Method C

A solution of Compound I corresponding to the saturation concentration at 70°C was prepared by dissolving Form N-1 in 20ml of nitroethane and heating the solution to 78°C. The solution was then cooled to 65°C. When the crystallizer temperature reached the desired temperature, the temperature was kept constant 24 hours. Nucleation was not observed. Temperature was then decreased to 64°C and after 24 hours to 63°C. Nucleation started after 4 days. This experiment was repeated three times and in all cases Form N-5 was formed.

### Example 2D: Stability of Form N-5

The stability of N-5 slurries in ethanol:water mixtures (1:1, 1:1.3 and 1:2) at 5, 25 and 30°C was determined after 1h, 48h and 5 days. Pure N-5 did not transform to any other form in any of these experiments. DSC and FTIR confirmed this.

## Claims

1. 3-[[3,5-dibromo-4-[4-hydroxy-3-(1-methylethyl)-phenoxy]-phenyl]-amino]-3-oxopropanoic acid in a crystalline form which is **characterised by** a powder X-ray diffraction pattern having major peaks at 2θ = 16.1±0.2. 20.1±0.2, 20.7±0.2, and 24.2±0.2.

2. A crystalline material as claimed in claim 1, **characterised by** an X-ray diffraction pattern additionally havng significant peaks at 20 = 8.9±0.2. 23.0±0.2. 25.9±0.2, 29.1±0.2, 29.4±0.2, and 30.2±0.2.

3. A crystalline material as claimed in claim 2, **characterised by** an X-ray diffraction pattern containing major peaks as fellows:
| 2-Theta | I% |
|---|---|
| 8.94 | 41.7 |
| 10.01 | 10.1 |
| 11.00 | 11.8 |
| 16.12 | 100.0 |
| 16.82 | 11.7 |
| 18.54 | 16.2 |
| 20.12 | 75.4 |
| 20.66 | 73.9 |
| 22.59 | 21.2 |
| 23.00 | 44.6 |
| 24.21 | 69.1 |
| 25.93 | 28.2 |
| 29.14 | 44.5 |
| 29.37 | 23.0 |
| 30.33 | 25.0 |

4. A crystalline material as claimed in any one of claims 1 to 3, which has a differential scanning calorimetry trace which exhibits a single endotherm with a maximum at 174±6°C.

5. A crystalline material as claimed in any one of claims 1 to 4, having a level of purity in which at least 90% of the 3-[[3,5-dibromo-4-[4-hydroxy-3-(1-methylethyl)-phenoxy]-phenyl]-amino]-3-oxopropanoic acid present, is in the required crystalline form.

6. A crystalline material as claimed in claim 5, having a level of purity in which at least 95% of the 3-[[3,5-dibromo-4-[4-hydroxy-3-(1-methylethyl)-phenoxy]-phenyl]-amino]-3-oxopropanoic acid present, is in the required crystalline form.

7. A crystalline material as claimed in claim 6, having a level of purity in which all of the 3-[[3,5-dibromo-4-[4-hydroxy-3-(1-methylethyl)-phenoxy]-phenyl]-amino]-3-oxopropanoic acid present, is in the required crystalline form.

8. A method for the preparation of a crystalline material as claimed in any one of claims 1 to 7, which comprises preparing a solution of 3-[[3,5-dibromo-4-[4-hydroxy-3-(1-methylethyl)-phenoxy]-phenyl]-amino]-3-oxopropanoic acid in a suitable solvent, and seeding with crystals of a crystalline material as claimed in any one of claims 1 to 7, under conditions such that the desired crystalline form is obtained.

9. A method as claimed in claim 8, in which the solvent is water, an alcohol, a ketone, DMSO, an ester, an acid, a nitrile, an amide, or a hydrocarbon; or a mixture thereof.

10. A method as claimed in claim 9, in which the solvent is an alcohol/water mixture.

11. A method as claimed in claim 10, which comprises preparing a solution of 3-[[3,5-dibromo-4-[4-hydroxy-3-(1-methylethyl)-phenoxy]-phenyl]-amino]-3-oxopropanoic acid in an alcohol or alcohol mixture; adding water; optionally filtering the resulting solution; and seeding the resulting solution with crystals of a material as claimed in any one of claims 1 to 7, to produce further crystals in the desired form.

12. A pharmaceutical composition comprising a crystalline material as claimed in any one of claims 1 to 7, together with a pharmaceutically acceptable carrier.

13. A crystalline material as claimed in any one of claims 1 to 7 or a composition as claimed in claim 12 for use in a method of therapy.

14. A crystalline material or a composition as claimed in claim 13, for use in the treatment of a condition associated with thyroid disfunction.

15. A crystalline material or a composition as claimed in claim 13, for use in the treatment of obesity, hypercholesterolemia, atherosclerosis, depression, osteoporosis, hypothyroidism or subclinical hypothyroidism, non-toxic goiter, papillary or follicular thyroid cancer, glaucoma, cardiovascular disease, congestive heart failure, and skin disorders.

## Patentansprüche

1. 3-[[3,5-Dibrom-4-[4-hydroxy-3-(1-methylethyl)-phenoxy]-phenyl]-amino]-3-oxopropansäure in kristalliner Form, die durch ein Pulver-Röntgenbeugungsdiagramm mit Hauptpeaks bei
2θ = 16,1 ± 0,2, 20,1 ± 0,2, 20,7 ± 0,2 und 24,2 ± 0,2
gekennzeichnet ist.

2. Kristallines Material nach Anspruch 1,
**gekennzeichnet durch** ein Röntgenbeugungsdiagramm, das außerdem deutliche Peaks bei 2θ = 8,9 ± 0,2, 23,0 ± 0,2, 25,9 ± 0,2, 29,1 ± 0,2, 29,4 ± 0,2 und 30,3 ± 0,2 aufweist.

3. Kristallines Material nach Anspruch 2,
**gekennzeichnet durch** ein Röntgenbeugungsdiagramm,
das Hauptpeaks wie folgt aufweist:
| 2-Theta | I (%) |
|---|---|
| 8,94 | 41,7 |
| 10,01 | 10,1 |
| 11,00 | 11,8 |
| 16,12 | 100,0 |
| 16,82 | 11,7 |
| 18,54 | 16,2 |
| 20,12 | 75,4 |
| 20,66 | 73,9 |
| 22,59 | 21,2 |
| 23,00 | 44,6 |
| 24,21 | 69,1 |
| 25,93 | 28,2 |
| 29,14 | 44,5 |
| 29,37 | 23,0 |
| 30,33 | 25,0 |

4. Kristallines Material nach einem der Ansprüche 1 bis 3,
das ein Diagramm der Differentialscanningkalorimetrie aufweist, das eine einzelne Endotherme mit einem Maximum bei 174 ± 6 °C zeigt.

5. Kristallines Material nach einem der Ansprüche 1 bis 4,
das einen Reinheitswert aufweist, bei dem mindestens 90 % der vorhandenen 3-[[3,5-Dibrom-4-[4-hydroxy-3-(1-methylethyl)-phenoxy]-phenyl]-amino]-3-oxopropansäure in der erforderlichen kristallinen Form vorliegen.

6. Kristallines Material nach Anspruch 5,
das einen Reinheitswert aufweist, bei dem mindestens 95 % der vorhandenen 3-[[3,5-Dibrom-4-[4-hydroxy-3-(1-methylethyl)-phenoxy]-phenyl]-amino]-3-oxopropansäure in der erforderlichen kristallinen Form vorliegen.

7. Kristallines Material nach Anspruch 6,
das einen Reinheitswert aufweist, bei dem die gesamte vorhandene 3-[[3,5-Dibrom-4-[4-hydroxy-3-(1-methylethyl)-phenoxy]-phenyl]-amino]-3-oxopropansäure in der erforderlichen kristallinen Form vorliegt.

8. Verfahren zum Herstellen eines kristallinen Materials nach einem der Ansprüche 1 bis 7,
das folgendes aufweist
- Herstellen einer Lösung von 3-[[3,5-Dibrom-4-[4-hydroxy-3-(1-methylethyl)-phenoxy]-phenyl]-amino]-3-oxopropansäure in einem geeigneten Lösungsmittel und
- Impfen mit Kristallen eines kristallinen Materials nach einem der Ansprüche 1 bis 7 unter derartigen Bedingungen, daß die gewünschte kristalline Form erhalten wird.

9. Verfahren nach Anspruch 8,
wobei das Lösungsmittel Wasser, ein Alkohol, ein Keton, DMSO, ein Ester, eine Säure, ein Nitril, ein Amid oder ein Kohlenwasserstoff oder ein Gemisch davon ist.

10. Verfahren nach Anspruch 9,
wobei das Lösungsmittel ein Alkohol/Wasser-Gemisch ist.

11. Verfahren nach Anspruch 10,
das das Herstellen einer Lösung von 3-[[3,5-Dibrom-4-[4-hydroxy-3-(1-methylethyl)-phenoxy]-phenyl]-amino]-3-oxopropansäure in einem Alkohol oder Alkoholgemisch, das Zugeben von Wasser, das wahlfreie Filtern der entstandenen Lösung und das Impfen der entstandenen Lösung mit Kristallen eines Materials nach einem der Ansprüche 1 bis 7 aufweist, um weitere Kristalle in der gewünschten Form zu erzeugen.

12. Pharmazeutische Zusammensetzung,
die ein kristallines Material nach einem der Ansprüche 1 bis 7 zusammen mit einem pharmazeutisch akzeptablen Träger aufweist.

13. Kristallines Material nach einem der Ansprüche 1 bis 7 oder Zusammensetzung nach Anspruch 12 für die Verwendung bei einem Therapieverfahren.

14. Kristallines Material oder Zusammensetzung nach Anspruch 13 für die Verwendung bei der Behandlung eines Zustandes, der mit einer Schilddrusenfehlfunktion in Zusammenhang steht.

15. Kristallines Material oder Zusammensetzung nach Anspruch 13 für die Verwendung bei der Behandlung von Fettsucht, Hypercholesterinämie, Atherosklerose, Depression, Osteoporose, Hypothyreose oder subklinischer Hypothyreose, nicht-toxischem Struma, papillärem oder follikulärem Schilddrüsenkrebs, Glaukom, Herzgefäßerkrankungen, dekompensierter Herzinsuffizienz (Stauungsinsuffizienz) und Hautleiden.

## Revendications

1. Acide 3-[[3,3-dibromo-4-[4-hydroxy-3-(1-méthyl éthyl)phénoxy]-phényl]-amino]-3-oxopropanoique sous une forme cristalline qui est **caractérisée par** un profil de diffraction des rayons X sur poudre ayant des pics majeurs à 2θ = 16,1±0,2, 20,1±0,2, 20,7±0,2, et 24,2±0,2.

2. Matériau cristallin selon la revendication 1, **caractérisé par** un profil de diffraction des rayons X ayant de plus des pics significatifs à 2θ = 8,9±0,2, 23,0±0,2, 25,9±0,2, 29,1±0,2, 29,4±0,2, et 30,3±0,2.

3. Matériau cristallin selon la revendication 2, **caractérisé par** un motif de diffraction des rayons X contenant les pics majeurs suivants :
| 2-thêta | I % |
|---|---|
| 8,94 | 41,7 |
| 10,01 | 10,1 |
| 11,00 | 11,8 |
| 16,12 | 100,0 |
| 16,82 | 11,7 |
| 18,54 | 16,2 |
| 20,12 | 75,4 |
| 20,66 | 73,9 |
| 22,59 | 21,2 |
| 23,00 | 44,6 |
| 24,21 | 69,1 |
| 25,93 | 28,2 |
| 29,14 | 44,5 |
| 29,37 | 23,0 |
| 30,33 | 25,0 |

4. Matériau cristallin selon l'une quelconque des revendications 1 à 3, qui a un tracé de calorimétrie différentielle à balayage qui présente une seule endothermie avec un maximum à 174±6°C.

5. Matériau cristallin selon l'une quelconque des revendications 1 à 4, ayant un niveau de pureté dans lequel au moins 90 % de l'acide 3-[[3,5-dibromo-4-[4-hydroxy-3-(1-méthyléthyl)phénoxy]-phényl]-amino]-3-oxopropanoïque présent sont sous la forme cristalline requise.

6. Matériau cristallin selon la revendication 5, ayant un niveau de pureté dans lequel au moins 95 % de l'acide 3-[[3,5-dibromo-4-[4-hydroxy-3-(1-méthyléthyl)-phénoxy]-phényl]-amino]-3-oxopropanoïque présent sont sous la forme cristalline requise.

7. Matériau cristallin selon la revendication 6, ayant un niveau de pureté dans lequel tout l'acide 3-[[3,5-dibromo-4-[4-hydroxy-3-(1-méthyléthyl)-phénoxy]-phényl]-amino]-3-oxopropanoïque présent est sous la forme cristalline requise.

8. Procédé pour la préparation d'un matériau cristallin tel que revendiqué dans l'une quelconque des revendications 1 à 7, qui comprend la préparation d'une solution d'acide 3-[[3,5-dibromo-4-[4-hydroxy-3-(1-méthyléthyl)-phénoxy]-phényl]-amino]-3-oxopropanoïque dans un solvant adapté, et l'ensemencement avec des cristaux d'un matériau cristallin tel que revendiqué dans l'une quelconque des revendications 1 à 7, dans des conditions telles que la forme cristalline souhaitée est obtenue.

9. Procédé selon la revendication 8, dans lequel le solvant est l'eau, un alcool, une cétone, le DMSO, un ester, un acide, un nitrile, un amide ou un hydrocarbure ; ou un de leurs mélanges.

10. Procédé selon la revendication 9, dans lequel le solvant est un mélange d'alcool et d'eau.

11. Procédé selon la revendication 10, qui comprend la préparation d'une solution d'acide 3-[[3,5-dibromo-4-[4-hydroxy-3-(1-méthyléthyl)-phénoxy]-phényl]-amino]-3-oxopropanoïque dans un alcool ou un mélange alcoolique ; l'addition d'eau ; éventuellement la filtration de la solution résultante ; et l'ensemencement de la solution résultante avec des cristaux d'un matériau tel que revendiqué dans l'une quelconque des revendications 1 à 7, afin de produire d'autres cristaux sous la forme souhaitée.

12. Composition pharmaceutique comprenant un matériau cristallin tel que revendiqué dans l'une quelconque des revendications 1 à 7, conjointement à un véhicule pharmaceutiquement acceptable.

13. Matériau cristallin selon l'une quelconque des revendications 1 à 7, ou composition selon la revendication 12, pour utilisation dans une méthode thérapeutique.

14. Matériau cristallin ou composition selon la revendication 13, pour utilisation dans le traitement d'un état associé à un dysfonctionnement de la thyroïde.

15. Matériau cristallin ou composition selon la revendication 13, pour utilisation dans le traitement de l'obésité, de l'hypercholestérolémie, de l'athérosclérose, de la dépression, de l'ostéoporose, de l'hypothyroïdie ou de l'hypothyroïdie infraclinique, du goitre non toxique, du cancer de la thyroïde papillaire ou folliculaire, d'un glaucome, d'une maladie cardiovasculaire, d'une insuffisance cardiaque congestive, et de troubles cutanés.
